**Europäisches Patentamt**

**European Patent Office**

**Office européen des brevets**

(19)

(11) Veröffentlichungsnummer: **0 025 226**
**B1**

(12)

# EUROPÄISCHE PATENTSCHRIFT

(45) Veröffentlichungstag der Patentschrift:
**20.03.85**

(21) Anmeldenummer: **80105326.5**

(22) Anmeldetag: **05.09.80**

(51) Int. Cl.⁴: **A 61 K 9/36,** A 61 K 9/28,
A 23 G 3/00, A 23 G 3/26

(54) **Dragierverfahren.**

(30) Priorität: **06.09.79 DE 2936040**

(43) Veröffentlichungstag der Anmeldung:
**18.03.81 Patentblatt 81/11**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
**FR - A - 1 222 582**
**FR - A - 1 337 719**
**FR - A - 1 537 651**
**FR - E - 77 963**
**US - A - 2 757 124**

**(Lactose) Zucker Süsswaren Wirtsch. (29)-32-36 1976 J.**
**Kube, B. Pritzwald Stegmann**

(73) Patentinhaber: **Meggle Milchindustrie GmbH & Co. KG,**
**Megglestrasse 6 - 12, D-8094 Reitmehring (DE)**

(72) Erfinder: **Boesig, Werner, Zugspitzstrasse 29,**
**D-8100 Garmisch-Partenkirchen (DE)**
Erfinder: **Pritzwald-Stegmann, Bernd, Föhrenstr.4,**
**D-8094Reitmehring (DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel**
**Postfach 860820, D-8000 München 86 (DE)**

# Beschreibung

Die Erfindung betrifft ein Verfahren zum Dragieren von Kernen, wie Pharmadragee-Kernen, Schokolinsen, Haselnüssen und dergleichen, mit einem Dragiermittel, im wesentlichen bestehend aus Saccharose, wenigstens einem weiteren Zukker und Wasser.

Dragierverfahren zum Überziehen von Tablettenkernen oder Pillen mit einer harten Zuckerschicht sind an und für sich bekannt; vgl. Ullmanns Enzyklopädie der Technischen Chemie, 3. Auflage, Band 4, Seite 10, und Band 19, Seite 260. Dabei wird die Zuckerschicht in Form eines flüssigen Sirups in den mit Dragee-Kernen gefüllten Dragierkessel gebracht. Durch das Drehen des Dragierkessels überziehen sich alle Drageekerne mit einem flüssigen Zuckerfilm. Sobald dies erfolgt ist (nach etwa 1 Minute), wird Warm- bzw. Kaltluft in den Kessel geblasen und der dünne Film wird fest. Dieser Vorgang wird je nach Wunsch 20 bis 50 oder gar 80mal wiederholt.

Bei den bekannten Dragierverfahren zum Überziehen von Kernen oder Pillen mit Zuckerdecken kommen als Dragiermittel üblicherweise wässrige Lösungen von Saccharose zum Einsatz, denen je nach Verwendungszweck noch Geschmacksstoffe, Geruchsstoffe, Farbstoffe und/oder Hilfsstoffe zugesetzt sind. Derartige Saccharoselösungen neigen im Verlauf des Dragierprozesses zum vorzeitigen Auskristallisieren. Im handwerklichen Verfahren umgeht man diese Schwierigkeit entweder dadurch, dass man die Lösung nacherwärmt, oder dadurch, dass man aus dem Vorratsbehälter einfach die klare Lösung von den Kristallen abschöpft und in den Dragierkessel gibt. Dabei verarmt aber die Dragierlösung um den Anteil des auskristallisierten Zuckers.

Bei modernen industriellen Verfahren wird der Dragiervorgang mehr oder weniger automatisiert. Wendet man ein kontinuierliches Verfahren an, so muss die Lösung aus dem Vorratsbehälter in den Dragierkessel gepumpt werden. Auf dem Wege vom Vorratsbehälter zum Dragierkessel kann sich die Lösung unter Umständen abkühlen und es besteht dann die Gefahr, dass Saccharose im Rohrsystem auskristallisiert. Die Bereinigung einer solchen Panne ist nur unter Demontage des Rohrsystems und durch Spülen möglich. Ein Auskristallisieren muss daher unter allen Umständen vermieden werden.

Gerade bei kontinuierlichen, industriellen Dragierverfahren gilt es daher, solche saccharosehaltigen Dragiermittel einzusetzen, aus denen auch im Falle einer gewissen Abkühlung keine Saccharose auskristallisiert. Bei den bekannten Dragierverfahren mit Dragiermitteln aus im wesentlichen Saccharose trat aber das unerwünschte Ereignis einer Kristallisation von Saccharose gelegentlich auf.

Erfindungsgemäss wird nun ein Dragierverfahren bereitgestellt, bei dem die Kristallisationsneigung von Saccharose ganz erheblich herabgesetzt ist.

Gegenstand der Erfindung ist somit ein Verfahren zum Dragieren von Kernen, wie Pharmadragee-Kernen, Schokolinsen, Haselnüssen und dergleichen, mit einem Dragiermittel, im wesentlichen bestehend aus Saccharose, wenigstens einem weiteren Zucker und Wasser, das dadurch gekennzeichnet ist, dass man die Kerne mit einer wässrigen Dragierlösung, bei welcher der weitere Zuckerbestandteil Lactose ist, in an sich bekannter Weise dragiert.

Bei einer bevorzugten Ausführungsform des erfindungsgemässen Verfahrens wird ein Dragiermittel eingesetzt, bei dem das Gewichtsverhältnis von Saccharose zu Lactose zwischen 90:10 und 50:50 liegt; bei einer besonders bevorzugten Ausführungsform liegt das Gewichtsverhältnis von Saccharose zu Lactose zwischen 80:20 und 70:30.

Die Dragierverfahren, die gemäss der Erfindung mit dem Dragiermittel durchgeführt werden können, sind an und für sich bekannt; vgl. z.B. Ullmann, Enzyklopädie der Technischen Chemie, 3. Auflage, Band 4, Seite 10, und Band 19, Seite 260. Das erfindungsgemässe Verfahren wird in an sich bekannter Weise mit einem Dragiermittel durchgeführt, das zusätzlich zu Saccharose noch Lactose als weiteren Zucherbestandteil enthält. Die Bezeichnung «im wesentlichen» bedeutet hier, dass das in den erfindungsgemässen Verfahren verwendete Dragiermittel neben den angegebenen Hauptbestandteilen Saccharose, Lactose und Wasser darüber hinaus auch noch relativ geringe Mengen an üblichen Zusatz- und Hilfsstoffen enthalten kann. So enthält das Dragiermittel, das im wesentlichen aus Saccharose, Lactose und Wasser besteht, zur Anpassung an den jeweiligen Verwendungszweck in der Regel auch noch übliche Geschmacks-, Geruchs- und Farbstoffe als weitere Bestandteile.

Als Geschmacks- und Geruchsstoffe kommen hier zum Beispiel Pfefferminzöl und insbesondere Vanillin in Betracht.

Geeignete Farbstoffe sind Lebensmittelfarbstoffe, wie Waldmeistergrün, Himbeerrot, Zitronengelb, Pistaziengrün, Kirschrot und andere. Diese Farbstoffe werden dem Dragiermittel gewöhnlich in Form ihrer verdünnten wässrigen Lösung zugesetzt.

Ein wichtiger Vorteil, den das Verfahren der Erfindung und das Dragiermittel zu seiner Durchführung bieten, ist die erheblich herabgesetzte Neigung, dass ein Zucker – gleichgültig, ob Saccharose und/oder Lactose – unter üblichen Dragierbedingungen auskristallisiert. Die erheblich geringere Kristallisationstendenz bei der praktischen Durchführung des erfindungsgemässen Kristallisationsverfahrens wird durch die Vergleichsversuche gemäss den Beispielen 1 bis 3 demonstriert. Bei Beispiel 4 wurden Saccharose/Wasser- und Saccharose/Lactose/Wasser-Lösungen abgestufter Zusammensetzungen von 108 °C auf Raumtemperatur abgekühlt und die Dauer bis zum Beginn der Kristallisation gemessen. Die Versuche zeigen, dass die Zusammensetzungen gemäss Tabelle 1 der Versuche mit 900 g Saccharose/100 g Lactose/350 ml $H_2O$ und 700 g Saccharose/300 g Lactose/350 ml $H_2O$ eine längere Dauer

bis zum Beginn der Kristallisation aufwiesen bzw. es trat überhaupt keine Kristallisation ein. Dass der Effekt, den Kristallisationsbeginn so hochkonzentrierter Saccharoselösungen um so viel länger hinauszuzögern, überhaupt möglich ist, und dass dieser Effekt gerade durch Zusatz des wenig wasserlöslichen Zuckers Lactose bewirkt wird, ist überraschend. Denn dieser Effekt tritt auf, obwohl Lactose im Vergleich zu Saccharose in Wasser sehr viel weniger löslich ist.

Ein weiterer Vorteil, der durch die Erfindung erreicht werden kann, ist darin zu sehen, dass der Geschmack der Drageehülle durch den Lactoseanteil beeinflusst wird. Es wird nämlich durch Ersatz eines Teils der Saccharose durch Lactose die Süsse herabgesetzt, obwohl die Zuckermenge dabei an sich gleich bleibt. Eine herabgesetzte Süsse von Dragees ist aber in manchen Fällen wünschenswert.

Ein weiterer Vorteil ist durch die Erfindung zu erreichen: im Unterschied zum üblichen Verfahren ist es nun möglich, den eigentlichen Dragierprozess, also das Überziehen des Kerns mit der Zuckermasse, bei wesentlich niedrigeren Temperaturen als üblich durchzuführen, was durch die verminderte Kristallisationsneigung des erfindungsgemässen Dragiermittels ermöglicht wird.

Die Beispiele erläutern die Erfindung.

Beispiel 1

5 kg Pharmadragee-Kerne (Lactose-Placebos aus 98,5% Lactose, 0,5% Magnesiumstearat, 1,0% Mikrozellulose) wurden in einem Dragierkessel mit einer maximalen Füllmenge von 10 kg bei 50%-iger tatsächlicher Füllmenge dragiert.

Das Dragiermittel aus 900 g Saccharose, 100 g Lactose, 350 ml Wasser und 10 ml wässriger Himbeerrotlösung wurde hergestellt, indem erst 100 g Lactose durch Erwärmen in 350 ml Wasser klar aufgelöst wurden und dann 900 g Saccharose, mittelfeine Körnung, zugegeben wurden. Nach kurzem Kochen bei 108 °C im offenen Kupferkessel war alles gelöst, und es wurde nach dem Abkühlen der Lösung auf 90 °C mit dem Dragieren begonnen.

Auf 5 kg Pharmadragee-Kerne wurden 100 ml der Dragiermittellösung aufgetragen. Die Trocknung des Zuckerlösungsfilms (Deckel) auf dem Dragee-Kern erfolgte mit heisser Luft von 40 °C. Nach ca. 5 Minuten war die Decke trocken und die zweite Decke wurde in Form einer weiteren 100 ml-Charge der Dragiermittellösung aufgegeben. Danach wurde wieder ca. 5 Minuten getrocknet. Ab der dritten Decke wurde mit reduzierter Flüssigkeitsaufgabemenge gearbeitet, nämlich 40 ml Dragiermittellösung für die zweite bis sechste Schicht, 45 ml für die siebte bis achte Schicht, 50 ml für die neunte bis elfte Schicht, 55 ml für die zwölfte bis vierzehnte Schicht und 45 ml, zuzüglich 10 ml heisses Wasser, für die fünfzehnte Schicht. Es war zu beachten, dass während der Flüssigkeitsaufnahme keine Warmluft geblasen wurde, damit die Oberfläche besser verschmiert. Der Dragiervorgang wurde in dieser beschriebenen Form bis zur Dragierdecke Nr. 15 durchgeführt. Bei der letzten Decke verdünnte man den Lösungsansatz etwas und erreichte dadurch eine etwas glattere Drageedecke. Nach dem Dragieren wurden die fertigen Dragees auf Hordenwagen zum Nachtrocknen ausgelegt.

Im Vorratsbehälter war keine Kristallisation aus der 90 °C heissen Dragiermittellösung festzustellen. Der Versuch wurde wiederholt mit einem geänderten Gewichtsverhältnis von Saccharose zu Lactose; nämlich 800 g Saccharose/200 g Lactose (1b), 700 g Saccharose/300 g Lactose (1c) und 500 g Saccharose/500 g Lactose (1d). Bei den Versuchen 1b und 1c trat ebenfalls keine Kristallisation ein, bei Versuch 1d erst nach einer Stunde.

Ein Vergleichsversuch mit einer Dragiermittellösung von 1000 g Saccharose allein, in gleicher Weise durchgeführt, ergab, dass die Kristallisation aus der 90 °C heissen Dragiermittellösung in dem Vorratsbehälter bereits nach 10 Minuten einsetzte.

Beispiel 2

15 kg Schokolinsen wurden analog Beispiel 1 hartdragiert. Davon abweichend wurde aber mit Luft von Raumtemperatur getrocknet, um die Schokolade nicht zum Schmelzen zu bringen und unter Verwendung eines Drageekessels mit 30 kg maximaler Füllmenge. Es wurden zwei Ansätze (Beispiel 2a und 2b) mit zwei verschiedenen Dragiermittellösungen gefahren. Die zwei eingesetzten Dragiermittellösungen aus

2a) 3,5 kg Saccharose,
    1,5 kg Lactose,
    1,7 l Wasser und
    10 ml Pfefferminzöl
bzw.
2b) 2,5 kg Saccharose,
    2,5 kg Lactose,
    1,7 l $H_2O$,
    30 ml wässrige Himbeerrotlösung und
    10 ml Pfefferminzöl
wurden wie in Beispiel 1 hergestellt.

Es wurden jeweils 80 Drageedecken aufgebracht. Am Schluss wurden in bekannter Weise drei Andeckschichten mit einer Lösung aus gequollener Gelatine, Saccharose und Wasser sowie eine letzte Schicht aus Sirup und Wasser aufgebracht.

Dabei erhielt man Schokolinsen mit einer relativ harten Aussenschicht aus Zucker.

Bei Beispiel 2a trat im Vorratsbehälter keine Kristallisation aus der heissen Dragiermittellösung ein; bei Beispiel 2b setzte nach 2,5 Stunden eine leichte Kristallisation ein.

Beispiel 3

13 kg bereits angedeckte Haselnüsse (geröstet, gesüsst, eingedickt) wurden weichdragiert. Weichdragierung heisst, dass man analog dem Hartdragieren eine Zuckerlösung (Dragiermittellösung) kocht, jedoch nach der Aufgabe einer bestimmten Lösung zum Produkt im Drageekessel die Decke nicht mit Warmluft trocknet, sondern mit Zucker, z.B. Saccharose oder auch Lactose, abstreut und die überflüssige Feuchtigkeit teilweise verdunsten und teilweise in den eingestreuten Zucker gehen lässt. Es wurden zwei Ansätze (Beispiel 3a und 3b) mit zwei verschiedenen Dragiermittellösungen gefahren. Die zwei Dragiermittellösungen aus

3a) 3,5 kg Saccharose,
   1,5 kg Lactose,
   1,7 l Wasser,
   50 ml wässriger Colabraunlösung und
   5 g Vanillin

bzw.

3b) 2,5 kg Saccharose,
   2,5 kg Lactose,
   1,7 l Wasser,
   50 ml wässriger Orangegelblich-Lösung und
   5 g Vanillin

wurden wie in Beispiel 1 hergestellt.

Es wurden jeweils zehn Decken aufgebracht, und jede Decke wurde unmittelbar nach der Aufbringung mit Saccharose abgestreut.

Bei Beispiel 3a trat keine Kristallisation aus der heissen Dragiermittellösung in dem Vorratsbehälter ein, bei Beispiel 3b kam es zu einer gegenüber konventionellen Verfahren verzögerten Kristallisation.

Beispiel 4

Die Bestandteile Saccharose/Wasser bzw. Saccharose/Lactose/Wasser in verschiedenen Mengenverhältnissen (vgl. Tabelle 1, Spalte 1) wurden bei 108 °C gekocht, bis alles gelöst war, und es wurde das Kristallisationsverhalten beim Abkühlen bzw. bei konstanter Temperatur untersucht.

In einem Becherglas liess man die heissen Lösungen unter Rühren (45 U/min) bzw. ohne Rühren auf Raumtemperatur, hier 26 °C, abkühlen. Dabei wurde beobachtet, ob bzw. wann eine Kristallisation eintrat. Die Versuche wurden längstens nach 20 Stunden abgebrochen.

Nach welcher Zeit, bei welcher Temperatur oder ob überhaupt eine Kristallisation einsetzte, ist in nachstehender Tabelle 1, Spalte 2 dargestellt.

1,350 kg jeder Lösung gemäss Spalte 1 der Tabelle 1 wurden nach der Herstellung durch Kochen bei 108 °C in einen Vorratsbehälter für Dragiermittellösung einer halbtechnischen Dragieranlage (V = 15 Liter) gebracht und die Lösung durfte sich dort abkühlen. Auch hier wurde innerhalb von 3 Stunden beobachtet, ob und gegebenenfalls nach wieviel Minuten die erste Kristallisation eintrat. Die Ergebnisse sind in Tabelle 1, Spalte 3 aufgeführt.

Beispiel 5

Dragierversuche unter Variation des Saccharose/Lactose-Verhältnisses

Pharmadragee-Kerne (Placebos aus 98,5% Lactose, 0,5% Magnesiumstearat und 1% Mikrocellulose) wurden in einer Anlage, wie in Beispiel 1 beschrieben, dragiert. Die Kesselneigung betrug 37 °C, die Kesseldrehzahl 30 upm. Die Temperatur der eingeblasenen Warmluft betrug 40 °C. Die Feuchtlaufzeit betrug eine Minute, die Trocknungszeit ca. 2,5 Minuten.

a) Versuch mit einer Zuckerlösung aus 80% Saccharose und 20% Lactose (800 g Saccharose/ 200 g Lactose/350 ml $H_2O$) bei einer Kochtemperatur von 108 °C: Die Lösung kristallisierte auch bei Abkühlung auf 20 °C nach 5 Stunden noch nicht aus. Die Dragierung verlief problemlos.

b) Versuch mit einer Zuckerlösung aus 70% Saccharose und 30 % Lactose (700 g Saccharose/ 200 g Lactose/350 ml $H_2O$) bei einer Kochtemperatur von 106 °C: Diese Lösung liess sich sehr gut verarbeiten. Nach 2-stündigem Dragierprozess zeigte sich im Vorratskessel noch keine Kristallisation des Sirups.

c) Vergleichsversuch mit einer reinen Saccharose-Lösung (1000 g Saccharose/350 ml $H_2O$) bei einer Kochtemperatur von 108 °C:

Die Lösung begann bei Abkühlung auf etwa 70 °C nach 10 Minuten auszukristallisieren. Ein Dragieren mit dieser Lösung war nur bedingt möglich.

Tabelle 1 – Das Kristallisationsverhalten beim Abkühlenlassen von der Kochtemperatur (108 °C) auf Raumtemperatur

| Bestandteile der Lösung | | | Laborversuch | | | | Betriebsversuch |
| Saccharose (g) | Lactose (g) | Wasser (ml) | unter Rühren | | ohne Rühren | | Beginn der Kristallisation (nach Minuten) |
| | | | Beginn der Kristallisation | | | | |
| | | | (Zeit, | Temp.) | (Zeit, | Temp.) | |
|---|---|---|---|---|---|---|---|
| 1000 | — (=Vergleich) | 350 | 20' | 67° | 35' | 55° | 10' stark |
| 900 | 100 | 350 | 65' | 47° | 90' | 40° | — |
| 800 | 200 | 350 | 300' | 29° | 960' | 27° | — |
| 700 | 300 | 350 | — | 26° | — | 26° | — |
| 600 | 400 | 350 | 30' | 58° | 30' | 58° | — |
| 500 | 500 | 350 | 30' | 58° | 30' | 58° | 60' |

**Patentansprüche**

1. Verfahren zum Dragieren von Kernen, wie Pharmadragee-Kernen, Schokolinsen und Haselnüssen mit einem Dragiermittel, im wesentlichen bestehend aus Saccharose, wenigstens einem weiteren Zucker und Wasser, dadurch gekennzeichnet, dass man die Kerne mit einer wässrigen Dragierlösung, bei welcher der weitere Zuckerbestandteil Lactose ist, in an sich bekannter Weise dragiert.

2. Verfahren nach Anspruch 1, dadurch gekennzeichnet, dass man nit einem Dragiermittel, bei dem das Gewichtsverhältnis von Saccharose zu Lactose zwischen 90:10 und 50:50 liegt, dragiert.

3. Verfahren nach Anspruch 2, dadurch gekennzeichnet, dass man mit einem Dragiermittel, bei dem das Gewichtsverhältnis von Saccharose zu Lactose zwischen 80:20 und 70:30 liegt, dragiert.

**Claims**

1. Process for the drageeing of cores, such as pharmaceutical dragee cores, chocolate drops and hazel nuts, with a drageeing agent consisting essentially of saccharose, at least one further sugar and water, characterised in that one dragees the cores in per se known manner with an aqueous drageeing solution in which the further sugar component is lactose.

2. Process according to claim 1, characterised in that one dragees with a drageeing agent in which the weight ratio of saccharose to lactose lies between 90:10 and 50:50.

3. Process according to claim 2, characterised in that one dragees with a drageeing agent in which the weight ratio of saccharose to lactose lies between 80:20 and 70:30.

**Revendications**

1. Procédé de dragéification de noyaux, tels que des noyaux de dragées pharmaceutiques, des lentilles de chocolat et des noisettes, avec un agent de dragéification, se composant essentiellement de saccharose, d'au moins un autre sucre et d'eau, caractérisé en ce qu'on dragéifie les noyaux d'une manière connue en soi avec une solution aqueuse de dragéification dans laquelle l'autre sucre constitutif est le lactose.

2. Procédé suivant la revendication 1, caractérisé en ce qu'on dragéifie avec un agent de dragéification dans lequel le rapport pondéral du saccharose au lactose est entre 90:10 et 50:50.

3. Procédé suivant la revendication 2, caractérisé en ce qu'on dragéifie avec un agent de dragéification dans lequel le rapport pondéral du saccharose au lactose est entre 80:20 et 70:30.